# EUROPEAN PATENT APPLICATION

(11) **EP 1 334 699 A1**
(43) Date of publication of application: **13.08.2003**
(21) Application number: 02425066.4
(22) Date of filing: 11.02.2002
(51) Int. Cl.: A61B 18/14

(54) **Apparatus for electrosurgery**

(71) Applicant: Led S.p.A., 03100 Frosinone (IT)
(72) Inventor: Mauti, Aido, 03100 Frosinone (IT); Mauti, Paolo, 03100 Frosinone (IT)
(74) Representative: Leone, Mario

(57) **Abstract**

Apparatus for electrosurgery (1) of a type apt to treat neoplastic tissues by thermoablation, comprising at least two pairs of mutually displaceable electrodes (4,5,6,7;41,51,61,71) and a generator (2,3) of electrosurgical current to make a thermoablation current flow between the electrodes according to a bipolar thermoablation mode.

## Description

The present invention refers to an apparatus for electrosurgery, and in particular to an apparatus for the treatment of neoplastic tissues by thermoablation.

One of the therapies for the cure of primary or metastatic tumours, above all at a hepatic and pulmonary level, consists in the electrosurgical thermoablation of the tumour tissue targeted by the treatment, a tissue typically consisting of a nodule.

The electrosurgical apparatuses for generating said thermoablative effect mainly consist of a generator of alternated and high-frequency current, delivered by means of a so-called active electrode. The latter is generally needle-shaped and placed percutaneously or with an open approach onto the target tumour tissue to be eliminated. The thermoablation is attained as, near to the active electrode, an elevated current density and therefore a substantial increase in temperature by Joule effect is generated, which in turn devitalizes the cells in a region surrounding the electrode itself.

The electrosurgical apparatuses at issue provide a so-called monopolar thermoablation, i.e. the circuit of the electrosurgical current delivered by the active electrode is closed via a so-called neutral or return electrode which has a very wide contact area with the patient's skin and is placed at a region of the patient's body totally separated from the region concerned by the actual surgery.

This operation modes in the tumour tissue thermoablation surgery entail several drawbacks.

One of the main drawbacks lies in that said modes merely enable the treatment of a very limited tissue portion at a time. In particular, the region concerned by the thermoablation is reduced to a small neighbourhood of the point of delivery of the current by the active electrode. This is also due to the fact that the dehydration which onsets near the active electrode impedes the continuation of flow of the thermoablation current.

The technical problem underlying the present invention is to provide an apparatus and a device for electrosurgery enabling to overcome the drawbacks hereto mentioned with reference to the known art.

This problem is solved by an apparatus for electrosurgery of the type apt to the treatment of neoplastic tissues by thermoablation, comprising:
- at least two pairs of electrodes; and
- current generating means for generating an electrosurgical current apt to make a thermoablation current flow between each pair of electrodes according to a bipolar thermoablation mode.

According to the same inventive concept, the present invention further relates to a device for electrosurgery apt to be applied onto a patient's body, comprising:
- one supporting body; and
- four electrodes, apt to be connected in pairs to current generating means for generating an electrosurgical current, which electrodes are constrained to said supporting body and arranged at the vertexes of a quadrilateral, said electrodes having an adjustable relative distance.

The present invention provides several relevant advantages.

A significant advantage lies in that the provision of a plurality of electrode pairs powered according to a bipolar thermoablation mode enables to widen the viable dimensions for the target region. Moreover, said electrode pairs and the bipolar powering mode thereof enable a great versatility in the extension and shape of the treatable tumour regions, in the selection of the route for the thermoablation currents and in the electrode placement.

Moreover, the device for electrosurgery of the invention enables to attain, at the central portion of the quadrilateral according to which the electrodes are placed, a constructive interference between the thermoablation currents delivered by the two electrode pairs, and therefore a sum of the intensities thereof. As it will be detailed hereinafter, this enables a reduction of the current intensity that each electrode has to deliver in order to attain the desired thermoablation. Thus, the dehydration of the tissues surrounding each electrode is reduced, and of course this promotes the flowing of the electrosurgical current in a region wider than that allowed by the abovedescribed known systems.

Other advantages, features and the modes of employ of the present invention will be made apparent in the following detailed description of some embodiments thereof, given as a non-limiting example. Reference will be made to the figures of the attached drawings, wherein:
- Figure 1 is a block diagram of a first embodiment of the apparatus for electrosurgery according to the present invention;
- Figure 2 is a block diagram of a second embodiment of the apparatus for electrosurgery according to the present invention;
- Figure 3 is an exploded perspective view of a device for electrosurgery according to the present invention;
- Figure 4 is a perspective view of the device of Figure 3 in an assembled configuration; and
- Figure 5 is a perspective view of a comb electrode, useful when combined with the apparatus for electrosurgery according to the present invention.

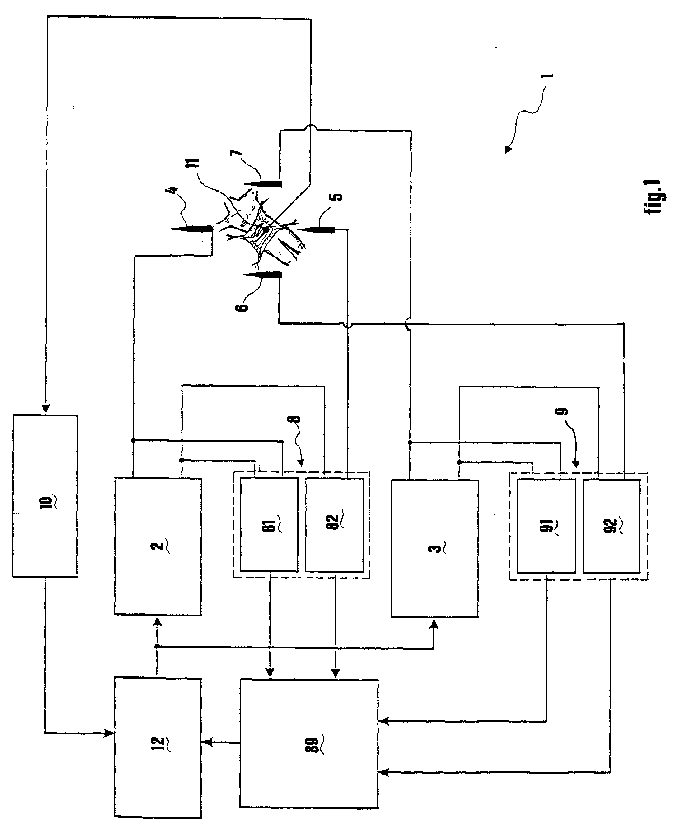

With initial reference to Figure 1, a first embodiment of the apparatus for electrosurgery according to the invention is generally indicated with 1. The apparatus 1 comprises means for generating an electrosurgical current, incorporated in an outside casing of the apparatus itself (not shown in the figures). In particular, in the present embodiment such means are made by a first and a second current generator, indicated with 2 and 3, respectively, electrically isolated the one from the other and apt to generate currents having substantially different frequencies. Due to reasons which will be made apparent hereinafter, these frequencies preferably differ of at least 100 kHz.

By way of example, the maximum power deliverable by each generator 2, 3 can be of about 75 W, on an impedance of about 200 Ohm seen therefrom.

Each of such generators 2 and 3 feeds a respective pair of electrodes according to a bipolar operation mode of each of such pairs. In particular, the apparatus 1 comprises a first and a second electrode, indicated with 4 and 5, powered from the first generator 2 and a third and fourth electrode, indicated with 6 and 7, respectively, and powered by the second generator 3.

These electrodes 4, 5, 6 and 7 can be permanently connected to the respective generator 2 or 3 or be removably connectable thereto by suitable ports provided at the outer casing of the apparatus 1. In the latter case, the casing containing the two generators 2 and 3 and the further components of the apparatus 1 which will be described could be provided to an end user separately from said four electrodes 4, 5, 6 and 7.

Preferably, each of the electrodes 4, 5, 6 and 7 has a coating of anti-stick material or it has been subjected to an anti-stick treatment at the related current delivering portion, in order to avoid the adhering of the treated tissue thereto.

Moreover, always according to a preferred embodiment, the electrodes 4, 5, 6 and 7 are made of Titanium and have the tip end electrically insulated or made of an insulating material, in order to avoid the need for cooling of the electrodes themselves or of the treated body region. The insulating tip prevents that the reduction of the electrode size in the zone thereof produces a current density such as to char the treated tissue.

Alternative embodiments can provide electrodes made of any metal sufficiently rigid to enable penetration into the body tissue without bending and capable of conducting heat so to remove that generated during the treatment near the electrode.

The apparatus 1 further comprises first and second impedance determining means, respectively combined with the first and to the second generator 2 and 3 and indicated with 8 and 9, respectively. Said means 8 and 9, analogous to the corresponding means already present in the electrosurgical apparatuses of the known art, provide each a current measuring unit, 81 and 91 respectively, apt to measure the current flowing in the circuit made by the respective pair of electrodes 4 and 5 or 6 and 7 and by the respective generator 2 or 3. This impedance determining means 8 and 9 further provide a voltage measuring unit, 82 and 92 respectively, apt to measure the voltage associated with said circuit across the respective generator 2 or 3.

The apparatus 1 further comprises an impedance computing unit 89 common to the first and second impedance determining means 8 and 9. The latter unit 89 is inputted the data related to the voltage and current measurements carried out by the voltage measuring units 82 and 92 and by the current measuring units 81 and 91, respectively, and it outputs the corresponding impedance values.

As it is known, this impedance determining is carried out both to determine the effect of the thermoablation treatment and for safety reasons, i.e. to control the regular current flow in the region concerned by the thermoablation.

It will be appreciated that the determining of the impedance between each pair of electrodes 4, 5 e 6, 7 enables to monitor the treatment uniformity.

The apparatus 1 further comprises a temperature measuring unit 10, which in turn provides a temperature sensor 11 apt to be applied at a central portion of the region concerned by the thermoablation in order to measure the local temperature thereof. As in the case of the electrodes 4, 5, 6 and 7, also the sensor 11 can be removably connectable to further components of the related unit 10 internal to the outside casing of the apparatus 1.

The apparatus 1 further comprises a control unit 12 for controlling the delivered power, connected to the temperature measuring unit 10 and to the impedance computing unit 89, apt to control the power delivered by the generators 2 and 3 as a function of the data outputted by said units 10 and 89. In particular, the control unit 12 is apt to modulate the strength of the current outputted by the generators 2 and 3 as a function of the actual temperature and impedance values, in order to prevent as much as possible damage to healthy tissues.

Since the impedance determining means 8 and 9, the temperature measuring unit 10 and the control unit 12 are made with hardware, and optionally with software, components and according to modes well-known to a person skilled in the art, a further description thereof will be omitted.

Hereinafter, the operation modes of the hereto described apparatus 1 will be illustrated.

As it is schematically shown in Figure 1, the electrodes 4, 5, 6 and 7 can be placed onto the neoplastic tissue to be eliminated at the vertexes of a quadrilateral, and in particular of a square, in this latter case being equidistant. Preferably, in this arrangement the electrodes of each pair 4, 5 or 6, 7 are placed at opposed vertexes of said quadrilateral.

Then, the two generators 2 and 3 are driven by the control unit 12 so as to make a respective alternated high-frequency thermoablation current flow between each pair of electrodes 4, 5 and 6, 7.

The abovedescribed quadrilateral arrangement and the bipolar thermoablation mode attained with each pair of electrodes 4, 5 and 6, 7 make the related thermoablation currents overlap at the central zone of the quadrilateral itself, i.e. at the central zone of the tumour tissue to be eliminated, substantially adding the respective intensities thereamong.

This interference between thermoablation currents can also be attained with an electrode arrangement according to the vertexes of a rhombus or of a rectangle, or even of a irregular quadrilateral in general in lieu of a square. However, in this latter case the thermoablation is advantageously more regular and homogeneous.

As abovementioned, said current interference entails several relevant advantages. These advantages will be better appreciated considering that in the known systems, near to the site of current delivering by the active electrode, there ensues an elevated temperature gradient, with the entailed charring of the tissues directly contacted by the electrode and with a marked dehydration of the tissues surrounding the latter which impedes the current to continue to flow. In order to prevent this excessive drying of the tissues surrounding the active electrode various contrivances have been devised, like, e.g., a slow heating of the active electrode apt to promote heat propagation, a forced water cooling thereof, or a cooling of the tissue by a flow of physiological water. However, even with these contrivances the diameter of the treated tumour can not be greater than about 2-3 cm .

Instead, in the present invention, by virtue of said current overlapping, the strength of the current flowing between each pair of electrodes 4, 5 and 6, 7 can be reduced with respect to the known systems, and therefore, with respect thereto, the overheating of the tissues surrounding the current delivery zone is reduced. This entails a lesser dehydration of the tissues near the electrodes and an improved loss of the heat produced, bringing the diameter of the concomitantly treatable tumour region to about 5 cm. All of these advantages are also attained by virtue of the fact that the coagulation and the dehydration begin at the central section of the treated region rather than near to the electrodes.

In short, the provision of a quadrilateral arrangement of the electrodes and of two generators insulated therebetween, each associated with a pair of opposed electrodes, enables to attain an interferential current which produces the maximum Joule effect not near to the electrodes but in the central area of the target zone defined thereby, and precisely at the intersection of the lines connecting the opposed electrodes powered in a bipolar mode. In this way, the extension of the ablative treatment is not impeded and the entire territory comprised within the quadrilateral defined by the electrodes can progressively be treated.

Furthermore, the distribution of the thermoablation current in the region to be treated is more homogeneous with respect to the known systems, and it is such that, in case an excessive drying of a specific zone occur, the bipolar current can flow in a neighbouring zone.

Moreover, of course to the reduction of the current strength delivered by each electrode and required for the thermoablation there is associated also a lowering of the required voltage thereof. This implies the further advantage, with respect to the known systems, of substantially limiting the phenomena of electromagnetic interference of the apparatus for electrosurgery 1 with monitoring apparatuses like ultrasound or radioscopy ones.

The fact that the two generators 2 and 3 be insulated the one from the other and have frequencies substantially different therebetween prevents the interference between the respective currents to trigger unwanted stimulations. In fact, frequencies not strictly equal thereamong could result in low frequency beats capable of producing a patient's neuromuscular or muscular stimulation. On the contrary, in case said frequencies differ thereamong of at least 100 kHz, the beating frequency will not be able to produce a stimulation, as observed in the known d'Ansorval's studies, but a mere Joule effect.

Moreover, another advantage lies in that the impedance computed by the impedance determining means 8 and 9 is less affected by phenomena of excessive drying about the electrodes, and therefore determinable with greater precision with respect to the known systems.

Further, the area external to that delimited by the electrodes can be considered as damage-free, as only marginally affected by the current, whereas the quadrilateral region delimited by the electrodes can be fully treated.

Returning now to the operation modes of the apparatus 1, the quadrilateral arrangement of the electrodes 4, 5 and 6, 7 enables, among other things, to position the temperature sensor 11 centrally to the treated tumour region, as it is schematically shown in Figure 1.

The control unit 12 can provide two distinct working modes, and precisely an operative mode, in which the thermoablation of neoplastic tissues is actually carried out, and a monitoring mode, in which weaker currents are made flow between the electrode pairs 4, 5 and 6, 7, in order to assess the actual value of the working impedance by means of said impedance determining means 8 and 9.

Of course, the bipolar mode delivery of current prevents the known problem of the possible burning of a patient's skin at the plate which typically implements the neutral electrode in a monopolar mode.

Furthermore, it will be appreciated that with the hereto described apparatus 1 two types of tumour treatment are viable, specifically, a treatment based on the devitalization of the actual neoplastic mass, and/or a treatment based on the devitalization of a layer of normal tissue surrounding the neoplastic mass, so as to have the neoplastic cells die by starving.

In particular, since at a 5 kHz frequency the impedance of the neoplastic tissue is about thrice that of the normal tissue, whereas at 1 MHz the ratio approaches 1:1, should it be desired to directly treat the neoplastic mass, the working frequencies of the two generators could be e.g., of 950 kHz and 1050 kHz, whereas should it be desired to 'line' the tumour devitalizing the surrounding healthy tissue, the frequencies at issue could be, e.g., of 350 kHz and 450 kHz, thereby restricting the current diffusion preferentially to the healthy tissue.

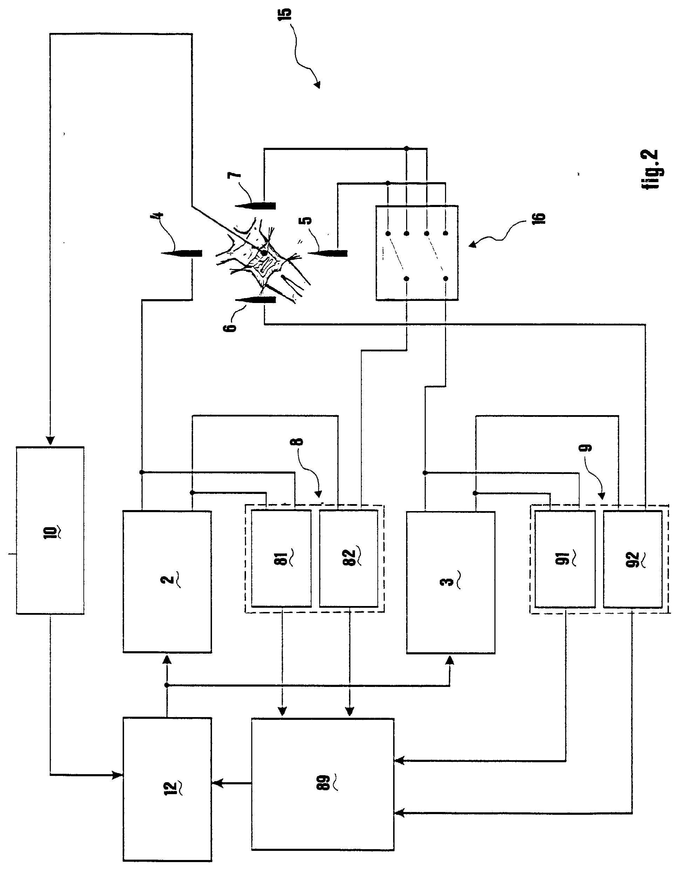

With reference now to Figure 2, an apparatus for electrosurgery of a second embodiment of the invention is generally indicated with 15.

Hereinafter, the apparatus 15 will be described merely with reference to the aspects differentiating it from the previous embodiment. Hence, components alike the abovedescribed ones are indicated by the same reference number. In particular, the apparatus 15 comprises a first and a second electrode 2 and 3 and two electrode pairs 4, 5 and 6, 7 in all analogous to the abovedescribed ones.

In a first working mode, the first and the second electrode 3 and 4 are connected to the first generator 2, whereas the third and the fourth generator 6 and 7 forming the other pair are connected to the second generator 3.

The apparatus 15 further comprises switching means 16 apt to change the generator 2, 3 to which the second and the fourth electrodes, 5 and 7, respectively, are connected. This switching enables a thermoablation current to flow not only between opposed electrodes, but also between electrodes placed at adjacent vertexes of the quadrilateral, thereby enabling to treat also the zone interposed between said adjacent electrodes.

However, the apparatus according to the invention hereto described with reference to two main embodiments is susceptible of several further variant embodiments, some of which are indicated hereinafter.

According to a first variant, the apparatus of the abovedescribed second embodiment provides a greater switching versatility, e.g. involving also the first and the third electrode 4 and 6.

A second variant provides instead the presence of a single generator rather than two, apt to power both electrode pairs according to the abovedescribed bipolar operation mode.

Moreover, a third variant provides a different impedance determination mode with respect to that provided in the electrosurgical systems known to the art. In particular, it is provided that each electrode of at least one of said pairs be made of two portions electrically insulatable the one from the other. In a first operative mode, both electrode portions concur to output the thermoablation current. Further, the abovedescribed control unit provides a second operative mode, definable as impedance determination mode, in which the two portions of each electrode are electrically insulated. In particular, in this latter mode a first portion of each electrode of the pair is comprised in a current measuring circuit which also comprises the respective generator, whereas a second portion of each electrode is used to meter the voltage across the two portions, totally independently from said current metering, by a high input-impedance system.

A person skilled in the art will understand that this impedance determination technique is borrowed from the body impedance measurement techniques.

Lastly, a fourth variant provides that the apparatus of the invention may also operate in monopolar mode, therefore being provided with means for switching from the bipolar to the monopolar operative mode, as well as of a suitable connection port for a plate-type neutral electrode.

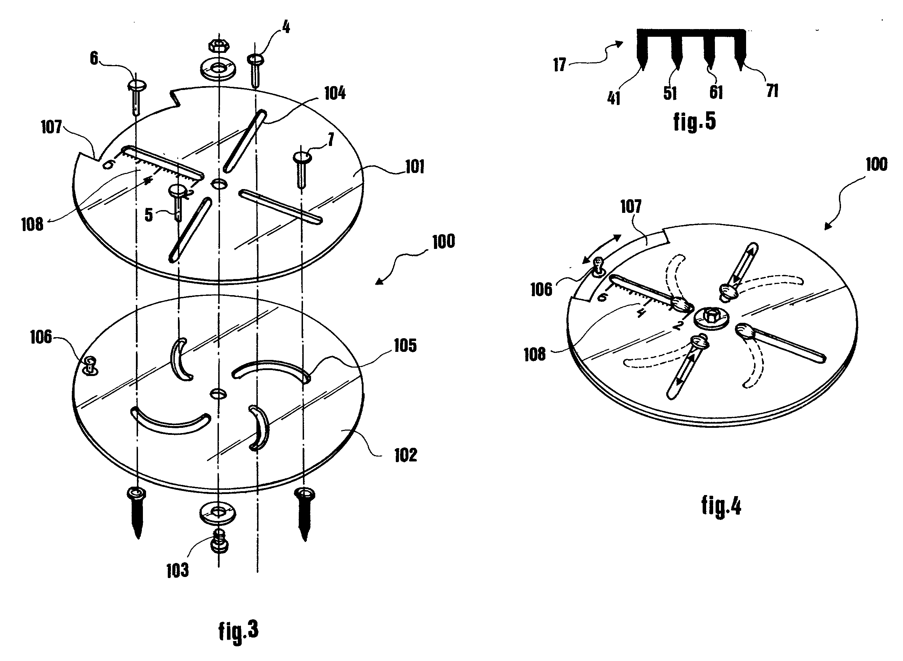

With reference to Figures 3 and 4, hereinafter a device for electrosurgery, generally indicated with 100, apt to be employed associated with the apparatus for electrosurgery according to the invention, and in particular with one of the abovedisclosed embodiments of these apparatuses will be described.

The device 100 comprises a supporting body made of two portions, and in particular formed by a first and a second body, 101 and 102 respectively, substantially flat and having the same disc-like shape. These bodies 101 and 102 are overlapped and movable, in particular rotatable, the one with respect to the other. To this end, they are connected by a central pin 103.

Preferably, the bodies 101 and 102 are made of Plexiglas.

The device 1 also comprises four electrodes, likewise indicated 4, 5, 6 and 7, in all analogous to the abovedescribed ones. These electrodes 4, 5, 6 and 7 are constrained to the two supporting bodies 101 and 102 and are apt to be connected in pairs to means for generating an electrosurgical current, it too analogous to the abovedescribed ones.

In particular, the electrodes 4, 5, 6 and 7 are placed at the vertexes of a quadrilateral, and in particular of a square, as seen above with reference to the arrangement of the electrodes of the apparatus for electrosurgery according to the invention.

Said electrodes 4, 5, 6 and 7 cross the bodies 101 e 102 through, and are held thereby by a shape coupling. The latter provides, for each electrode 4, 5, 6 or 7, a pair of grooves 104 and 105, obtained onto the first and the second body 101 and 102, respectively, and having a substantially rectilinear and a substantially arcuate development, respectively. The arrangement of the grooves 104 and 105 of each pair is such that those are overlapped for an extension such as to allow reception of the respective electrode therethrough.

Said grooves 104 and 105 implement means for adjusting the relative distance between the electrodes 4, 5, 6 and 7. In fact, by moving the bodies 101 and 102 the one with respect to the other, the electrodes 4, 5, 6 and 7 are guided to move in a nearing/moving away direction which depends on the verse of the relative motion between said bodies.

The particular shape and placement of the grooves 104 and 105 are such that the relative interelectrode distances always remain in a predetermined ratio, and in particular that the electrodes remain equidistant the one from the other.

The device 100 also comprises stopping means for limiting the excursion of the relative rotation between the bodies 101 and 102. These means consist of a engaging member 106 fixed to the second body 102, apt to abut onto the edges of a notch 107 obtained at a peripheral edge portion of the first body 101.

Lastly, the device 100 provides at least one graduation 108 apt to indicate to a user the relative distance between said electrodes.

It will be understood that variant embodiments can provide a different implementation of said means for adjusting the relative interelectrode distance.

With reference now to Figure 5, the apparatus according to the invention can also be employed combined with a device 17 for electrosurgery of the so-called 'comb' type and already known to a person skilled in the art. This latter device 17 comprises a plurality of electrodes, each corresponding to a respective comb tooth. In the present embodiment, it is provided the employ of a device having four electrodes, indicated with 41, 51, 61 and 71, respectively.

These four electrodes 41, 51, 61 and 71 are connected to the current generating means of the apparatus for electrosurgery according to the invention so as to form two contiguous electrode pairs 41, 51 and 61, 71, each apt to operate according to a bipolar thermoablation mode. In particular, according to a first embodiment variant, the device 17 is connected to an apparatus comprising a single electrosurgical current generator. In that case, each pair of contiguous electrodes 41, 51 and 61, 71 is connected to such single generator for the power-feeding in said bipolar mode.

According to a second variant, when the apparatus comprises two current generators, as in the case of the apparatus of the abovedescribed preferred embodiments of the invention, each of said contiguous electrode pairs 41, 51 and 61, 71 will be connected to a respective generator.

Lastly, in the light of the above, it will be understood that the invention implements a novel method of thermoablation of neoplastic tissues based on the following steps:
- providing at least two pairs of electrodes according to what has been disclosed hereto;
- providing means for generating an electrosurgical current of the hereto illustrated type; and
- making an electrosurgical current flow between each pair of electrodes according to a bipolar thermoablation mode.

Preferably, the thermoablation currents for the two electrode pairs have a substantially different frequency, in the light of the aboveillustrated with reference to the first embodiment of the apparatus of the invention.

Always as aboveillustrated, the invention enables to carry out two tumour treatment types, i.e. a treatment based on the devitalization of the actual neoplastic mass and/or a treatment based on the devitalization of a layer of healthy tissue surrounding the latter, so as to starve the tumour cells to death.

According to a preferred implementation of the method at issue, the electrodes of said pairs are placed onto a patient's body at the vertexes of a quadrilateral, so as to have the thermoablation currents flow between opposed or adjacent electrodes, as hereto illustrated.

The present invention has hereto been described according to preferred embodiments thereof. It is understood that there could be other embodiments referable to the same inventive concept, all however falling within the protective scope of the claims set forth hereinafter.

## Claims

1. A device for electrosurgery (100) apt to be applied onto a patient's body, comprising:
- one supporting body (101, 102); and
- four electrodes (4, 5, 6, 7), apt to be connected in pairs to current generating means for generating an electrosurgical current, which electrodes are constrained to said supporting body and arranged at the vertexes of a quadrilateral, said electrodes having an adjustable relative distance.

2. The device (100) according to claim 1, wherein said electrodes (4, 5, 6, 7) are constrained to said supporting body (101, 102) so that the relative distances thereof remain in a predetermined ratio.

3. The device (100) according to claim 2, wherein said electrodes (4, 5, 6, 7) are constrained to said supporting body (101, 102) so as to remain equidistant.

4. The device (100) according to claim 2 or 3, wherein said supporting body is made of two parts, comprising a first (101) and a second (102) body overlapped and movable the one with respect to the other, and wherein each of said electrodes (4, 5, 6, 7) is constrained to said first and second body by a shape coupling such that by moving said first and second body the one with respect to the other, the electrode is guided to move in a nearing/moving away direction, said direction depending on the sense of the relative motion between said bodies.

5. The device (100) according to claim 4, wherein said first (101) and second (102) body are rotatable the one with respect to the other.

6. The device (100) according to claim 5, wherein said shape connection provides, for each electrode (4, 5, 6, 7), a pair of grooves (104, 105) obtained onto the first (101) and second (102) body, respectively, the arrangement of the grooves of each pair being such that those are overlapped for an extension such as to allow reception of the respective electrode therethrough.

7. The device (100) according to claim 6, wherein said pair of grooves consists of a first groove (104) having a substantially rectilinear development and of a second groove (105) having a substantially arcuate development.

8. The device (100) according to any one of the claims 4 to 7, comprising stop means (106, 107) for limiting the excursion of the relative motion between said first (101) and second (102) body.

9. The device (100) according to any one of the claims 4 to 8, wherein said first (101) and second (102) body are made of Plexiglas.

10. The device (100) according to any one of the preceding claims, wherein said supporting body (101, 102) has at least one graduation (108) apt to indicate to a user the relative distance between said electrodes (4, 5, 6, 7).

11. An apparatus for electrosurgery (1) of the type apt to the treatment of neoplastic tissues by thermoablation, comprising:
- at least two pairs of electrodes (4, 5, 6, 7; 41, 51, 61, 71); and
- current generating means (2, 3) for generating an electrosurgical current apt to make a thermoablation current flow between each pair of electrodes according to a bipolar thermoablation mode.

12. The apparatus (1) according to claim 11, wherein said generating means (2, 3) comprises a single generator of electrosurgical current.

13. The apparatus (1) according to claim 11, wherein said generating means comprises two generators (2, 3) of electrosurgical current electrically isolated the one from the other, each apt to make a thermoablation current flow between the electrodes of a respective pair (4, 5; 6, 7).

14. The apparatus (1) according to claim 13, wherein said generators (2, 3) have frequencies substantially different the one from the other.

15. The apparatus (1) according to the preceding claim, wherein said frequencies differ of at least 100 kHz.

16. The apparatus (1) according to any one of the claims 13 to 15, comprising switching means (16) apt to change the generator (2, 3) to which at least two electrodes (5, 7) of said pairs are connected.

17. The apparatus (1) according to any one of the claims 11 to 16, comprising an electrosurgical device (100) according to any one of the claims 1 to 10.

18. The apparatus (1) according to any one of the claims 11 to 16, comprising a comb-shaped electrosurgical device (17), wherein each electrode (41, 51, 61, 71) of said pairs is implemented by a respective tooth of said comb-shaped device.

19. The apparatus (1) according to any one of the claims 11 to 18, wherein each electrode (4, 5, 6, 7) of at least one pair of said electrode pairs is made of a first and of a second part electrically insulatable the one from the other, and wherein the apparatus comprises impedance determining means apt to enable a current measurement in a circuit comprising said first electrode parts and said current generating means and a voltage measurement, independent from said current measurement, across said second electrode parts.

20. The apparatus (1) according to any one of the claims 11 to 19, wherein said electrodes (4, 5, 6, 7) have an anti-stick coating.
